# EUROPÄISCHE PATENTANMELDUNG

(11) **EP 3 616 658 A1**
(43) Veröffentlichungstag der Anmeldung: **04.03.2020**
(21) Anmeldenummer: 19193047.8
(22) Anmeldetag: 22.08.2019
(51) Int. Cl.: A61F 2/958

(54) **VERFAHREN UND VORRICHTUNG ZUM EINBETTEN EINES IMPLANTATS IN EINE BALLONOBERFLÄCHE DURCH INDUKTIVES ERWÄRMEN DES IMPLANTATS**

(30) Priorität: 28.08.2018 DE 102018120940
(71) Anmelder: BIOTRONIK AG, 8180 Bülach (CH)
(72) Erfinder: Fargahi, Amir, 8180 Bülach (CH)
(74) Vertreter: Galander, Marcus

(57) **Zusammenfassung**

Die Erfindung betrifft ein Verfahren zum Fixieren eines Implantats (1) auf einen Ballon (2), wobei der Ballon (2) zusammen mit einem Implantat (1), der auf eine Ballonoberfläche (2a) des Ballons (2) gecrimpt ist, so dass das Implantat (1) mit einer Innenseite (1a) einen Kontaktbereich (21) die Ballonoberfläche (2a) kontaktiert, in einem Innenraum (7) einer Form (3) bereitgestellt wird, und wobei der Balloninnenraum (6) mit einem Druck beaufschlagt wird und das Implantat (1) induktiv erwärmt wird, so dass der Kontaktbereich (21) über das Implantat (1) erwärmt und plastisch verformt wird, wobei die Innenseite (1a) des Implantats (1) in die Ballonoberfläche (2a) eingebettet wird. Weiterhin betrifft die Erfindung eine durch das Verfahren hergestellte Anordnung (11) sowie eine Vorrichtung (10) zur Durchführung des Verfahrens.

## Beschreibung

Die Erfindung betrifft ein Verfahren und eine Vorrichtung zum Fixieren eines Implantats, bevorzugt eines Stents, auf einem Ballon eines Ballonkatheters sowie eine Anordnung mit einem auf einem Ballon fixierten Implantat sowie ferner eine Vorrichtung zur Durchführung des Verfahrens. Die vorliegende Erfindung wird am Beispiel eines Implantats und eines Ballons eines Ballonkatheters beschrieben. Die vorliegende Erfindung ist jedoch prinzipiell zur Fixierung eines beliebigen Implantates auf einem Ballon geeignet. Derartige Implantate sind beispielsweise Implantats, Herzklappenprothesen mit einem implantatartigen Grundgerüst, Okkluder oder allgemein rohrförmige, ballonexpandierbare Implantate

Bei auf Ballons eines Ballonkatheters gecrimpten Implantats ist es von hoher Wichtigkeit, dass das Implantat sicher auf dem jeweiligen Ballon eines Ballonkatheters fixiert sind, damit es beim Implantieren nicht bezüglich des Ballons verrutscht. Es ist daher wichtig, eine ausreichende Implantathaltekraft zu erzielen. Diese Implantathaltekraft stellt sich bei einen auf einen Ballon gecrimpten Implantat als Summe aus Formschluss- und Kraftschlussverbindungen dar.

In den einschlägigen Normen (z. B. ASTM Norm F2394-07) wird - so auch im Rahmen der vorliegenden Erfindung - unter einem Crimpen eines Implantats/Stents auf einen Ballon ein Sichern des Implantats auf dem Ballon verstanden, bei dem das Implantat auf dem Ballon in radialer Richtung (zum gefalteten Ballon hin) komprimiert und dabei plastisch deformiert wird.

Im Hinblick auf die Implantathaltekraft, entstehen formschlüssige Verbindungen durch das Ineinandergreifen von mindestens zwei Verbindungspartnern (hier Implantat und Ballon). Dadurch können sich die Verbindungspartner auch ohne oder bei unterbrochener Kraftübertragung nicht lösen. Anders ausgedrückt ist bei einer formschlüssigen Verbindung der eine Verbindungspartner dem anderen Verbindungspartner im Weg.

Kraftschlüssige Verbindungen setzen eine Normalkraft auf die miteinander zu verbindenden Flächen voraus. Ihre gegenseitige Verschiebung wird verhindert, solange die durch die Haftreibung bewirkte Gegenkraft nicht überschritten wird. Der Kraftbeziehungsweise Reibschluss ist verloren und die Flächen rutschen aufeinander, wenn die tangential wirkende Lastkraft größer als die Haftreibungskraft ist.

In der US 9,566,371 wird vorgeschlagen, das Implantat zu erhitzen und den Ballon unter Druck zu setzen, um den Ballon mit dem Implantat zu verschweißen. Die WO 2017/011200 beschreibt ein Erhitzen und Komprimieren eines medizinischen Implantats.

Hiervon ausgehend liegt der vorliegenden Erfindung die Aufgabe zugrunde, eine präzise Fixierung eines auf einen Ballon gecrimpten Implantats bezüglich des Ballons bereitzustellen, um insbesondere das Risiko des Verrutschens des Implantats, insbesondere während einer Implantation, zu verhindern. Insbesondere wird eine Methode zur präziseren und schnelleren Temperatursteuerung bei der Fixierung gesucht

Diese Aufgabe wird durch ein Verfahren mit den Merkmalen des Anspruchs 1, eine Anordnung mit den Merkmalen des Anspruchs 14 sowie durch eine Vorrichtung mit den Merkmalen des Anspruchs 15 gelöst.

Ausführungsformen dieser Erfindungsaspekte sind in den entsprechenden Unteransprüchen angegeben und/oder werden nachfolgend beschrieben.

Gemäß Anspruch 1 wird ein Verfahren zum Fixieren eines Implantats auf einen Ballon (für einen Ballonkatheter) offenbart, wobei der Ballon, der einen mit Druck beaufschlagbaren Balloninnenraum aufweist, zusammen mit einem Implantat, das auf eine Ballonoberfläche des Ballons gecrimpt ist, so dass das Implantat mit einer Innenseite einen Kontaktbereich der Ballonoberfläche kontaktiert, in einem Innenraum einer Form bereitgestellt wird, und wobei der Balloninnenraum mit einem Druck beaufschlagt wird und das Implantat induktiv erwärmt wird, so dass der Kontaktbereich des Ballons mit dem Implantats erwärmt und plastisch verformt wird, wobei die Innenseite des Implantats in die Ballonoberfläche eingebettet wird.

Der besagte Kontaktbereich muss nicht notwendigerweise ein zusammenhängendes Gebiet bilden, sondern kann sich auch aus mehreren separaten Kontaktstellen zusammensetzen. Unter Kontaktbereich wird im Rahmen der Anmeldung der Teil des Ballons verstanden, der in direktem Kontakt mit der Struktur des Implantats steht.

Das erfindungsgemäße Verfahren eignet sich besonders zum Fixieren eines Stents auf einem Ballon eines Ballonkatheters. Unter Stent wird dabei im Rahmen dieser Anmeldung eine dauerhafte oder degradierbare rohrförmige Struktur verstanden, die in ein Körpergefäß, insbesondere ein Blutgefäß, implantiert werden kann. Der Stent kann dabei auch ein oder mehrere, insbesondere wirkstoffhaltige, Beschichtungen aufweisen. Derartige Stent weisen vorteilhafterweise eine Vielzahl von Streben auf, die die Struktur des Stents bilden, wobei die Streben zumeist mäanderförmig verlaufen und als Vielzahl von Ringen und/oder Helizes angeordnet und derart miteinander verbunden sind, dass sie eine im Wesentlichen zylindrische Struktur mit einer Vielzahl von Zwischenräumen bzw. Durchgangsöffnungen zwischen den Stegen bilden.

Zur induktiven Erwärmung des Implantats ist vorzugsweise vorgesehen, dass das Implantat metallisch ist oder zumindest eine metallische Komponente aufweist.

Das Implantat (oder vorzugsweise der Stent) weist insbesondere eine Vielzahl an Durchgangsöffnungen auf, die sich jeweils von der Innenseite des Implantats zu einer Außenseite des Implantats erstrecken, wobei die jeweilige Durchgangsöffnung einen umlaufende laterale Wandung aufweist, über die die Innenseite des Implantats mit der Außenseite des Implantats verbunden ist, so dass durch das Einbetten der Innenseite des Implantats in die Ballonoberfläche ein der jeweiligen Durchgangsöffnung zugeordneter Bereich des Ballons bzw. der Ballonoberfläche in die jeweilige Durchgangöffnung hineinragt, wobei ein Formschluss zwischen dem Ballon und dem Implantat erzeugt wird. Hierbei schmiegt sich die Ballonoberfläche insbesondere auch zumindest abschnittsweise an die Wandung bzw. Begrenzung der jeweiligen Durchgangsöffnung an.

Die Durchgangsöffnungen des Implantats werden auch als Zellen des Implantats bezeichnet. Die jeweilige Zelle bzw. Durchgangsöffnung wird dabei jeweils vom Implantat berandet, z. B. durch miteinander verbundene Streben des Implantats. Die Streben können dabei einstückig bzw. integral miteinander verbunden sein. Insbesondere kann das Implantat auf diese Weise eine Gitterstruktur ausbilden. Ein solches Implantat kann z. B. durch entsprechendes Bearbeiten eines rohrförmigen (vorzugsweise metallischen) Vorformlings gebildet werden, wobei bei der Bearbeitung die Zellen bzw. Durchgangsöffnungen in das Implantat z. B. mittels Laser geschnitten werden, so dass Streben mit den Zellen (Durchgangsöffnungen) erzeugt werden. Es ist aber auch denkbar, die Zellen/Durchgangsöffnungen des Implantats auf andere Weise zu erzeugen.

Die plastische Verformung des besagten erhitzten Kontaktbereichs wird insbesondere dadurch erzielt, dass im Balloninnenraum ein Druck anliegt und eine das Implantat umgebende Innenseite der Form ein Widerlager für das Implantat bildet, d. h., die Außenseite des Implantats kann die Innenseite der Form kontaktieren bzw. gegen diese drücken.

Gemäß einer Ausführungsform des erfindungsgemäßen Verfahrens ist vorgesehen, dass das Implantat mittels eines Induktors induktiv erwärmt wird. Bei dem Induktor kann es sich z. B. um einen wendelförmigen Induktor, einen Gabelinduktor oder einen Klappinduktor handeln. Andere Induktordesigns sind auch denkbar. Bevorzugt ist der Induktor weiterhin gemäß einer Ausführungsform der Erfindung reinraumtauglich ausgebildet

Der Induktor kann z. B. eine Leistungsaufnahme im Bereich von 2kW aufweisen sowie eine Betriebsfrequenz, die z. B. im Bereich von 70 kHz bis 450 kHz liegt.

Weiterhin ist gemäß einer Ausführungsform des erfindungsgemäßen Verfahrens vorgesehen, dass die Form aus einem elektrisch nicht-leitfähigen Material besteht. Insbesondere kann die Form eines der folgenden Materialien aufweisen oder kann aus einem der folgenden Materialien bestehen: einem Glas, einer Keramik oder einem wärmeresistenten Kunststoff wie zum Beispiel PEEK (Polyetheretherketon), Teflon etc.

Weiterhin ist gemäß einer Ausführungsform des erfindungsgemäßen Verfahrens vorgesehen, dass das Implantat bzw. der besagte Kontaktbereich bei dem induktiven Erwärmen auf eine Soll-Temperatur erwärmt wird.

Weiterhin ist gemäß einer Ausführungsform des erfindungsgemäßen Verfahrens vorgesehen, dass eine Ist-Temperatur des Implantats beim Erwärmen des Implantats auf die Soll-Temperatur laufend oder wiederholt gemessen wird und die Ist-Temperatur des Implantats (beim Erwärmen des Implantats) auf die Soll-Temperatur geregelt wird.

Weiterhin ist gemäß einer Ausführungsform des erfindungsgemäßen Verfahrens vorgesehen, dass die Ist-Temperatur des Implantats beim Erwärmen des Implantats auf die Soll-Temperatur laufend oder wiederholt mit Hilfe einer Messung einer vom Implantat abgegebenen Wärmestrahlung gemessen wird. Die Temperaturmessung erfolgt also mit anderen Worten berührungslos. Insbesondere wird die Wärmestrahlung bzw. Infrarotstrahlung mit einem Pyrometer gemessen, um die Temperatur des Implantats zu bestimmen.

Weiterhin ist gemäß einer Ausführungsform des erfindungsgemäßen Verfahrens vorgesehen, dass die Form eine Durchgangsöffnung für die Transmission der zu messenden Wärmestrahlung aufweist.

Dabei kann die Durchgangsöffnung einen Durchmesser im Bereich von z. B. 0,3 mm bis 0,5 mm aufweisen.

Weiterhin ist gemäß einer Ausführungsform des erfindungsgemäßen Verfahrens vorgesehen, dass der besagte Druck im Balloninnenraum im Bereich von 10 bar bis 30 bar liegt und insbesondere 15 bar beträgt.

Weiterhin ist gemäß einer Ausführungsform des erfindungsgemäßen Verfahrens vorgesehen, dass die Soll-Temperatur in einem der folgenden Bereiche unterhalb der Schmelztemperatur liegt:
- im Bereich von 40°C bis 150°C,
- im Bereich von 40°C bis 140°C (insbesondere für den Fall, dass der Ballon aus PA 12 besteht, siehe unten),
- im Bereich von 60°C bis 150°C (insbesondere für den Fall, dass der Ballon aus Pebax 7033 besteht, siehe unten),
- im Bereich von 50°C bis 110°C,
- im Bereich von 100°C bis 110°C, insbesondere 102°C bis 107°C (insbesondere für den Fall, dass es sich bei dem Material des Ballons um Pebax 7033 handelt und/oder für den Fall eines metallischen Implantats oder Stents, auf den insbesondere kein Medikament aufgebracht ist),
- im Bereich von 50°C bis 60°C, insbesondere 53°C bis 57°C (insbesondere für den Fall, dass es sich bei dem Material des Ballons um PA 12 handelt und/oder dass auf dem Implantat oder Stent ein Medikament aufgebracht ist, d. h. es sich insbesondere um einen sogenannten Drug Eluting Stent (DES) handelt, d. h., um einen Stent, der dazu konfiguriert ist, ein Medikament abzugeben, im Gegensatz zu einem unbeschichtet Stent (BMS)).

Weiterhin ist gemäß einer Ausführungsform des erfindungsgemäßen Verfahrens vorgesehen, dass der Ballon aus einem Ballonmaterial gefertigt ist oder ein Ballonmaterial aufweist, insbesondere an der Ballonoberfläche, das eine Glasübergangstemperatur aufweist, wobei die Soll-Temperatur größer oder gleich der Glasübergangstemperatur ist, und wobei insbesondere die Soll-Temperatur nicht mehr als 10%, insbesondere nicht mehr als 5%, insbesondere nicht mehr als 1% von der Glasübergangstemperatur abweicht. Die Glasübergangstemperatur kann mit einer dem Fachmann bekannten Methode bestimmt werden, wie beispielsweise thermoanalytischen Methoden (DSC, DMA, DIL, LFA).

Gemäß einer Ausführungsform der vorliegenden Erfindung besteht der Ballon aus einem Material wie z. B. Polyamid oder Modifikationen davon, wie z. B. einem Polyether-Block - Amid (z. B. PEBAX®). Weiterhin kann es sich bei dem Material um ein thermoplastisches Elastomer, z. B. TPE-A, handeln. Diese teilkristallinen Kunststoffe (viele gebräuchliche Kunststoffe weisen einen kristallinen Anteil von 10% bis 80% auf) besitzen sowohl eine Glasübergangstemperatur unterhalb derer die amorphe Phase einfriert (einhergehend mit Versprödung), als auch eine Schmelztemperatur, bei der sich die kristalline Phase auflöst. Die Schmelztemperatur trennt den entropieelastischen Bereich deutlich vom Fließbereich.

Gemäß einer Ausführungsform der vorliegenden Erfindung kann der Ballon insbesondere aus einem der folgenden Materialien gefertigt sein bzw. bestehen: einem Polyamid, insbesondere PA 12, z. B. Grilamid Polyamid-12 L25; einem Polyether-Block-Amid (Peba), z. B. PEBAX® 3533 oder PEBAX® 7033; PET; PEEK; TPU.

Bei Grilamid Polyamid-12 L25 handelt es sich insbesondere um eine teilkristalline Thermoplaste mit einer Glasübergangstemperatur von 37°C sowie mit einer Schmelztemperatur von 178°C. Bei Peba bzw. PEBAX® 3533 (CAS-Nr. 77402-38-1) oder PEBAX® 7033 (CAS-Nr. 77402-38-1) handelt es sich um teilkristalline thermoplastische Elastomere (TPE), wobei z. B. PEBAX® 3533 eine Glasübergangstemperatur von -65°C und eine Schmelztemperatur von 144°C aufweist. Weiterhin kann PET (Polyethylenterephthalat) z. B. eine Glasübergangstemperatur von 70°C sowie eine Schmelztemperatur von z. B. 255°C aufweisen. Bei PEEK (Polyetheretherketon) handelt es handelt es sich insbesondere ebenfalls um eine teilkristalline Thermoplaste mit einer Glasübergangstemperatur von z. B. 143°C und einer Schmelztemperatur von z. B. 340°C. Weiterhin können im Rahmen der vorliegenden Erfindung thermoplastisches Polyurethan (TPU) als Material für den Ballon verwendet werden.

Weiterhin ist gemäß einer Ausführungsform des erfindungsgemäßen Verfahrens vorgesehen, dass das Implantat oder der Stent bei mit dem Druck beaufschlagten Balloninnenraum über einen Zeitraum von zumindest 10 s, insbesondere zumindest 20 s, insbesondere zumindest 30 s, insbesondere zumindest 40 s, insbesondere zumindest 50 s, insbesondere zumindest 60s der besagten Soll-Temperatur ausgesetzt wird.

Gemäß einer Ausführungsform des Verfahren ist vorgesehen, dass der Ballon bei mit dem Druck beaufschlagten Balloninnenraum und bei dem im Innenraum der Hülle angelegten Unterdruck über einen Zeitraum von zumindest 10 s bis 100 s, insbesondere 20 s bis 80 s, insbesondere 30 s bis 60 s, insbesondere 20 s bis 40 s, insbesondere 25 s bis 35 s, insbesondere 50 s bis 70 s, insbesondere 55 s bis 65 s, der besagten Soll-Temperatur ausgesetzt wird.

Die Zeiträume 20 s bis 40 s bzw. 25 s bis 35 s, werden insbesondere verwendet, wenn es sich bei dem Implantat um einen Stent handelt, der dazu konfiguriert ist ein Medikament abzugeben (DES). Hierbei kann der Ballon z. B. aus PA 12 oder einem anderen Polyamid bestehen.

Die Zeiträume 50 s bis 70 s bzw. 55 s bis 65 s, werden insbesondere verwendet, wenn es sich bei dem besagten Implantat um einen rein metallischen Stent oder Implantat (BMS) handelt. Hierbei kann der Ballon z. B. aus PEBAX® 7033 oder einem sonstigen TPE, insbesondere TPE-A, bestehen.

Im Hinblick auf die verschiedenen Stent-Konfigurationen (DES oder BMS) oder entsprechenden Implantaten kann die vorliegende Erfindung insbesondere mit den folgenden beispielhaften Parametern durchgeführt werden:

| Stent/Implantat | Ballonmaterial | Endtemperatur °(C) | Zeitraum bzw. Prozessdauer (s) | Druck im Balloninnenraum (bar) |
|---|---|---|---|---|
| BMS | Pebax 7033 | 105+/- 3 | 60 | 15 +/- 0.5 |
| DES | PA 12 | 55+/- 2 | 30 | 15 +/- 0.5 |

Weiterhin ist gemäß einer Ausführungsform des erfindungsgemäßen Verfahrens vorgesehen, dass der Balloninnenraum mit dem Druck beaufschlagt wird, bevor das Implantat bzw. der besagte Kontaktbereich erwärmt wird. Die Beaufschlagung mit dem Druck kann jedoch auch während des Erwärmens des Implantats vorgenommen bzw. eingeleitet werden, oder nachdem das Implantat auf die Soll-Temperatur erwärmt worden ist.

Weiterhin betrifft die vorliegende Erfindung eine Anordnung, aufweisend ein Implantat, das auf eine Ballonoberfläche eines Ballons gecrimpt ist (insbesondere eines Ballons eines Ballonkatheters), wobei das Implantat mittels des Verfahrens nach einem der vorhergehenden Ansprüche an dem Ballon fixiert ist.

Weiterhin betrifft ein Aspekt der vorliegenden Erfindung eine Vorrichtung zum Fixieren eines Implantats an einem Ballon. Diese Vorrichtung eignet sich insbesondere zur Durchführung des erfindungsgemäßen Verfahrens und wird bevorzugt bei dem erfindungsgemäßen Verfahren verwendet.

Die erfindungsgemäße Vorrichtung weist zumindest auf:
- eine Form, die einen Innenraum zur Aufnahme eines auf einen Ballon gecrimpten Implantats aufweist,
- eine Einrichtung, die mit einem Balloninnenraum des Ballons in Strömungsverbindung bringbar ist und dazu ausgebildet ist, den Balloninnenraum des Ballons mit einem Druck zu beaufschlagen (z. B. durch Einleiten von Druckluft), und
- einen Induktor, der dazu konfiguriert ist, das Implantat induktiv bzw. berührungslos zu erwärmen, wenn der Ballon und das darauf gecrimpte Implantat im Innenraum der Form angeordnet sind.

Die besagte Form kann zum Beispiel zumindest abschnittsweise zylindrisch ausgebildet sein und kann dabei eine umlaufende Wandung aufweisen, die dazu ausgebildet ist, das Implantat zu umgeben, wenn dieser zusammen mit dem Ballon in dem Innenraum der Form angeordnet ist. Hierbei begrenzt die besagte Wandung den Innenraum und weist eine Innenseite auf, die dem Innenraum der Form zugewandt ist. Die Innenseite dient insbesondere als Anlagefläche bzw. Widerlager für das Implantat. Das Ballonmaterial bzw. die Ballonoberfläche kann somit durch Anlegen eines Drucks im Balloninnenraum gegen das Implantat bzw. dessen Innenseite drücken, wobei das Implantat durch die Innenseite der Form in radialer Richtung in zurückgehalten wird.

Gemäß einer Ausführungsform der erfindungsgemäßen Vorrichtung ist vorgesehen, dass die Vorrichtung einen Temperatursensor zum Messen einer momentanen Temperatur (d. h. Ist-Temperatur) des Implantats aufweist, wobei der Temperatursensor insbesondere dazu ausgebildet ist, die Temperatur mit Hilfe einer Messung einer durch das Implantat abgegebenen Wärmestrahlung zu messen, regulieren und Konstant halten. Der Temperatursensor kann z. B. als Pyrometer ausgestaltet sein.

Weiterhin ist gemäß einer Ausführungsform der erfindungsgemäßen Vorrichtung vorgesehen, dass die Form eine Durchgangsöffnung aufweist, die Wärmestrahlung vom Implantat zum Temperatursensor durchlässt, wobei die Durchgangsöffnung insbesondere zwischen dem Implantat und dem Temperatursensor angeordnet ist, und zwar insbesondere in der umlaufenden Wandung der Form.

Weitere Merkmale und Ausführungsformen der vorliegenden Erfindung sollen nachfolgend im Zusammenhang mit den Figuren beschrieben werden. Es zeigen:
- Fig. 1: eine schematische Darstellung einer Vorrichtung die zur Durchführung des erfindungsgemäßen Verfahrens verwendbar ist;
- Fig. 2: eine schematische Schnittdarstellung des am Ballon zu fixierenden Stents; und
- Fig. 3: eine perspektivische Ansicht eines Induktors einer Vorrichtung zur Durchführung des erfindungsgemäßen Verfahrens, wobei der Induktor die Form der Vorrichtung wendelförmig umgibt.

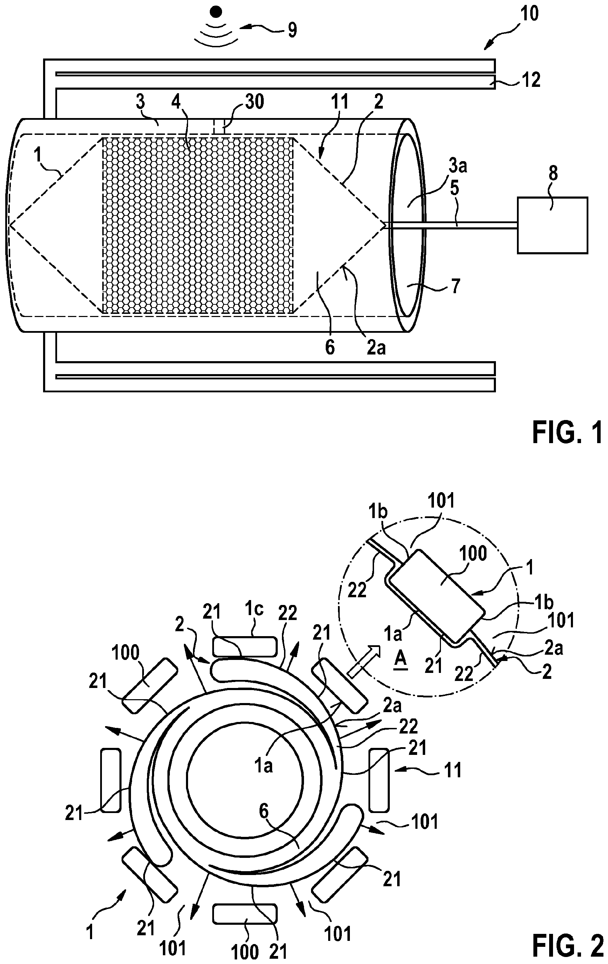

Figur 1 zeigt im Zusammenhang mit Figur 2 eine Ausführungsform einer Vorrichtung 10, die zur Durchführung des erfindungsgemäßen Verfahrens konfiguriert ist. Die Vorrichtung 10 weist danach eine z. B. zylindrische Form 3 mit einer Innenseite 3a auf, die einem Innenraum 7 der Form 3 zugewandt ist. Die Innenseite 3a bildet eine Anlagefläche für einen im Innenraum 7 anordenbaren Stent 1 aus, der auf eine Ballonoberfläche 2a eines insbesondere gefalteten Ballons 2 gecrimpt ist und mittels der Vorrichtung 10 zusätzlich in die Ballonoberfläche 2a eingebettet werden soll, um einen Formschluss zu erzeugen, der die Stenthaltekraft erhöht. Die Vorrichtung 10 verfügt weiterhin insbesondere über eine Einrichtung 8, die mit einem Balloninnenraum 6 des Ballons 2 in Strömungsverbindung bringbar ist und zum Beaufschlagen des Balloninnenraumes 6 des Ballons 2 mit einem Druck konfiguriert ist. Die Einrichtung kann z. B. als Druckgas- insbesondere Druckluftquelle 8 ausgestaltet sein, die dazu konfiguriert ist ein Gas, insbesondere Druckluft, in den Balloninnenraum 6 einzuleiten, um den besagten Druck im Balloninnenraum 6 zu erzeugen.

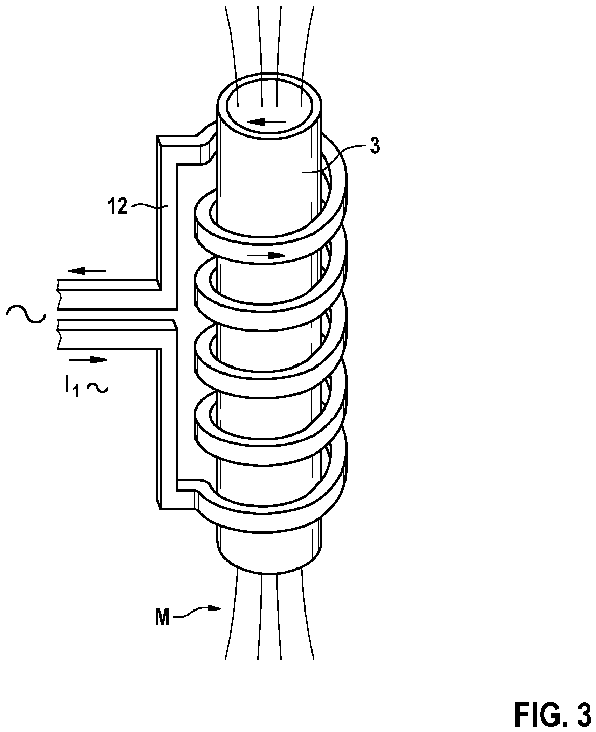

Weiterhin weist die Vorrichtung 10, wie es in der Figur 3 beispielhaft gezeigt ist, zumindest einen Induktor 12 auf, der dazu konfiguriert ist, den Stent 1 direkt, und zwar berührungslos zu erwärmen, wenn der Ballon 2 und der darauf gecrimpte Stent 1 im Innenraum 7 der Form 3 angeordnet sind. Der Induktor 12 ist dazu ausgebildet, ein magnetisches Feld M zu erzeugen, das im Stent 1 einen elektrischen Strom erzeugt, der wiederum Joulsche Wärme generiert, die den Stent 1 erwärmt. Der Induktor 12 kann gemäß Figur 3 wendelförmig ausgebildet sein, wobei sich der Induktor 12 wendelförmig um die Form 3 herum erstreckt, so dass der darin angeordnete Stent 1 mittels des Induktors 12 erwärmbar ist. Anstelle eines wendelförmigen Induktors 12 können auch andere, dem Fachmann bekannte Induktoren eingesetzt werden, z. B. ein Gabelinduktor oder ein Klappinduktor. Bei einem Gabelinduktor ist der Leiter des Induktors so angeordnet, dass der Induktor eine offene Seite aufweist an der man den zu erwärmenden Gegenstand so bezüglich des Induktors anordnen kann, dass sich der Induktor auf zwei einander abgewandten Seiten des Gegenstandes erstreckt. Ein Klappinduktor erlaubt das Öffnen des Induktors über ein Gelenk, so dass der Gegenstand im offenen Zustand des Klappinduktors im Induktor platzierbar ist und bei geschlossenem Induktor von diesem umgeben ist.

Durch das direkte Erwärmen des Stents 1 erwärmt dieser die Ballonoberfläche 2a bzw. ein entsprechendes Ballonmaterial lediglich in einem Kontaktbereich 21 zwischen einer Innenseite 1a des Stents 1 und der Ballonoberfläche 2a.

Der Stent 1 bzw. der Kontaktbereich 21 wird dabei so erhitzt, dass dieser plastisch verformbar wird. Durch den angewendeten Druck im Balloninnenraum 6 wird der Stent 1 mit seiner Innenseite 1a in die Ballonoberfläche eingedrückt bzw. eingebettet.

Außerhalb des Kontaktbereichs 21 wird der Ballon nicht bzw. in wesentlich geringerem Ausmaß erwärmt, so dass das Material außerhalb des Kontaktbereichs zumindest teilweise in einem plastisch nicht-verformbaren Zustand verbleiben kann.

Wie anhand der Figur 2 weiterhin ersichtlich ist, kann der Stent 1 eine Vielzahl an Durchgangsöffnungen 101 aufweisen, die sich jeweils von der Innenseite 1a des Stents 1 zu einer Außenseite 1c des Stents 1 erstrecken, wobei die jeweilige Durchgangsöffnung 101 eine durch den Stent 1 gebildete umlaufende laterale Wandung bzw. Begrenzung 1b aufweist, über die die Innenseite 1a des Stents 1 mit der Außenseite 1c des Stents 1 verbunden ist, so dass durch das Einbetten der Innenseite 1a des Stents 1 in die Ballonoberfläche 2a ein der jeweiligen Durchgangsöffnung 101 zugeordneter Bereich 22 des Ballons 2 bzw. der Ballonoberfläche 2a in die jeweilige Durchgangsöffnung 101 hineinragt (in der schematischen Darstellung gemäß Figur 2 ist dies durch Pfeile angedeutet), wobei ein Formschluss zwischen dem Ballon 2 und dem Stent 1 erzeugt wird. Hierbei schmiegt sich die Ballonoberfläche 2a insbesondere auch zumindest abschnittsweise an die laterale Wandung bzw. Begrenzung 1b der jeweiligen Durchgangsöffnung 101 an, wie insbesondere in dem Detail A der Figur 2 dargestellt ist. Die Durchgangsöffnungen 101 können jeweils insbesondere durch Streben 100 des Stents 1 begrenzt sein, die dann auch die besagten lateralen Wandungen bzw. Begrenzungen 1b bilden.

Um bei dem erfindungsgemäßen Verfahren die Temperatur des Stents 1 einstellen bzw. regeln zu können, ist insbesondere vorgesehen, die Temperatur mittels eines Temperatursensors 9 zu messen, wobei es sich bei dem Temperatursensor insbesondere um einen Temperatursensor handelt, der zur Bestimmung der Temperatur des Stents 1 dazu ausgebildet ist, eine vom Stent 1 ausgesendete Wärmestrahlung (z. B. Infrarotstrahlung) zu messen. Hierfür kann eine Wandung der Form 3 eine Durchgangsöffnung 30 aufweisen, die ein ungehindertes Austreten von Wärmestrahlung des Stents 1 erlaubt, derart, dass diese durch den Temperatursensor 9 erfassbar und zur Bestimmung der Ist-Temperatur des Stents 1 auswertbar ist. Der Temperatursensor 9 ist vorzugsweise zur Regelung der Temperatur des Stents 1 über eine nicht gezeigte Kontrolleinheit mit dem Induktor 12 verbunden.

Durch die erfindungsgemäße Lösung wird somit im Ergebnis eine optimale Formschlussverbindung zwischen Stent 1 und Ballon 2 erzeugt, die die Stenthaltekraft erhöht, wobei insbesondere das Ballonmaterial im Wesentlichen nur in dem Kontaktbereich 21 mit dem Stent 1 warm und dehnbar wird. Der Stent 1 kann hierdurch tief im Ballonmaterial eingebettet werden. Somit verringert sich in vorteilhafter Weise das Risiko eines Verschiebens des Stents (vgl. "Stent Displacement" gemäß ASTM F2394-07) sowie das Risiko eines Lösens des Stents vom Ballon (vgl. "Stent Dislodgment" gemäß ASTM F2394-07).

## Patentansprüche

**1.** Verfahren zum Fixieren eines Implantats (1) an einem Ballon (2), wobei der Ballon (2), zusammen mit einem Implantat (1), das auf eine Ballonoberfläche (2a) des Ballons (2) gecrimpt ist, so dass der Implantat (1) mit einer Innenseite (1a) einen Kontaktbereich (21) der Ballonoberfläche (2a) kontaktiert, in einem Innenraum (7) einer Form (3) bereitgestellt wird, und wobei ein Balloninnenraum (6) des Ballons mit einem Druck beaufschlagt wird und das Implantat (1) induktiv erwärmt wird, so dass der Kontaktbereich (21) des Ballons mit dem Implantat (1) erwärmt und plastisch verformt wird, wobei die Innenseite (1a) des Implantats (1) in die Ballonoberfläche (2a) eingebettet wird.

**2.** Verfahren nach Anspruch 1, dass das Implantat (1) mittels eines Induktors (12) induktiv erwärmt wird.

**3.** Verfahren nach einem der vorhergehenden Ansprüche, wobei die Form (3) aus einem elektrisch nicht-leitfähigen Material besteht.

**4.** Verfahren nach einem der vorhergehenden Ansprüche, wobei die Form (3) eines der folgenden Materialien aufweist oder aus einem der folgenden Materialien besteht: ein Glas, eine Keramik oder ein wärmeresistenter Kunststoff.

**5.** Verfahren nach einem der vorhergehenden Ansprüche, wobei das Implantat (1) bei dem induktiven Erwärmen auf eine Soll-Temperatur erwärmt wird.

**6.** Verfahren nach Anspruch 5, wobei eine Ist-Temperatur des Implantats (1) beim Erwärmen des Implantats (1) gemessen wird und die Ist-Temperatur des Implantats (1) beim Erwärmen des Implantats (1) auf die Soll-Temperatur geregelt wird.

**7.** Verfahren nach Anspruch 6, wobei die Ist-Temperatur des Implantats (1) beim Erwärmen des Implantats (1) mit Hilfe einer Messung einer vom Implantat (1) abgegebenen Wärmestrahlung gemessen und reguliert wird.

**8.** Verfahren nach Anspruch 7, wobei die Form (3) eine Durchgangsöffnung (30) für eine Transmission der zu messenden Wärmestrahlung aufweist.

**9.** Verfahren nach einem der vorhergehenden Ansprüche, wobei der Druck im Balloninnenraum (6) im Bereich von 10 bar bis 30 bar liegt.

**11.** Verfahren nach einem der Ansprüche 5 bis 9, wobei die Soll-Temperatur in einem der folgenden Bereiche liegt:
- im Bereich von 40°C bis 150°C,
- im Bereich von 40°C bis 140°C,
- im Bereich von 60°C bis 150°C,
- im Bereich von 50°C bis 110°C,
- im Bereich von 100°C bis 110°C, insbesondere 102°C bis 107°C,
- im Bereich von 50°C bis 60°C, insbesondere 53°C bis 57°C.

**12.** Verfahren nach einem der Ansprüche 5 bis 11, wobei der Ballon (2) aus einem Ballonmaterial gefertigt ist, das eine Glasübergangstemperatur aufweist, wobei die Soll-Temperatur größer oder gleich der Glasübergangstemperatur ist, und wobei insbesondere die Soll-Temperatur nicht mehr als 10%, insbesondere nicht mehr als 5%, insbesondere nicht mehr als 1% von der Glasübergangstemperatur abweicht.

**13.** Verfahren nach einem der Ansprüche 5 bis 12, wobei der Implantat (1) bei mit dem Druck beaufschlagten Balloninnenraum (6) über einen Zeitraum von zumindest 10 s bis 100 s, insbesondere 20 s bis 80 s, insbesondere 30 s bis 60 s, insbesondere 20 s bis 40 s, insbesondere 25 s bis 35 s, insbesondere 50 s bis 70 s, insbesondere 55 s bis 65 s, der besagten konstanten Soll-Temperatur ausgesetzt wird.

**14.** Anordnung (11), aufweisend einen Implantat (1), der auf eine Ballonoberfläche (2a) eines Ballons (2) gecrimpt ist, wobei der Implantat (1) mittels des Verfahrens nach einem der vorhergehenden Ansprüche an dem Ballon (2) fixiert ist.

**15.** Vorrichtung (10) zum Fixieren eines Implantats (1) an einem Ballon (2), mit:
- einer Form (3), die einen Innenraum (7) zur Aufnahme eines auf einen Ballon (2) gecrimpten Implantats (1) aufweist,
- einer Einrichtung (8), die mit einem Balloninnenraum (6) des Ballons (2) in Strömungsverbindung bringbar ist und zum Beaufschlagen des Balloninnenraumes (6) des Ballons (2) mit einem Druck konfiguriert ist, und
- einem Induktor (12), der dazu konfiguriert ist, das Implantat (1) zu erwärmen, wenn der Ballon (2) und das darauf gecrimpte Implantat (1) im Innenraum (7) der Form (3) angeordnet sind.
